# EUROPÄISCHE PATENTSCHRIFT

(11) **EP 3 088 022 B1**
(45) Veröffentlichungstag und Bekanntmachung des Hinweises auf die Patenterteilung: **30.09.2020**
(21) Anmeldenummer: 16164974.4
(22) Anmeldetag: 21.04.2010
(51) Int. Cl.: A61M 5/14, A61M 1/36

(54) **LUFTABSCHEIDER, EXTERNE FUNKTIONSEINRICHTUNG, BLUTKREISLAUF SOWIE BEHANDLUNGSVORRICHTUNG**
AIR SEPARATOR, EXTERNAL FUNCTIONAL DEVICE, BLOOD CIRCUIT AND TREATMENT DEVICE
SEPARATEUR D'AIR, DISPOSITIF DE FONCTIONNEMENT EXTERNE, DISPOSITIF DE TRAITEMENT ET DE CIRCULATION SANGUINE

(30) Priorität: 23.04.2009 DE 102009018664; 10.06.2009 DE 102009024465; 10.06.2009 US 185608 P
(43) Veröffentlichungstag der Anmeldung: 02.11.2016
(62) Teilanmeldung aus: 10716488.1
(73) Patentinhaber: Fresenius Medical Care Deutschland GmbH, 61352 Bad Homburg (DE)
(72) Erfinder: Brückner, Christoph, 97422 Schweinfurt (DE); Lauer, Martin, 66606 St. Wendel (DE); Weis, Manfred, 66606 St. Wendel (DE)
(74) Vertreter: Bobbert & Partner Patentanwälte PartmbB

(56) Entgegenhaltungen:
- DE-A1- 10 224 750
- JP-A- 2000 270 709
- US-A- 5 591 251
- US-A1- 2005 261 619

## Beschreibung

Die vorliegende Erfindung betrifft einen Luftabscheider gemäß Anspruch 1. Sie betrifft ferner eine externe Funktionseinrichtung gemäß Anspruch 11, ein Schlauchsystem zum extrakorporalen Führen von Blut (hierin auch als Blutkreislauf bezeichnet) gemäß Anspruch 13 sowie eine Behandlungsvorrichtung gemäß Anspruch 14.

Bei extrakorporal gefördertem Blut besteht die Gefahr der Bildung von Lufteinschlüssen oder Luftblasen. In der Regel kann es sinnvoll oder vielmehr notwendig sein, die Luft aus dem Blut zu entfernen bzw. abzuscheiden, z. B. bevor das Blut in den Patienten rückgeführt wird.

Die US 5,591,251 offenbart einen Blasenfänger und entsprechende Verfahren.

Aus der DE 102 24 750 A1 ist eine Vorrichtung zur Behandlung einer medizinischen Flüssigkeit bekannt.

Aus der US 2005/0261619 A1 ist eine Vortex-Luftabscheidung in einem Blutperfusionssystem bekannt.

Aufgabe der vorliegenden Erfindung ist, einen zu diesem Zweck geeigneten Luftabscheider anzugeben.

Diese Aufgabe wird beispielsweise durch einen Luftabscheider mit den Merkmalen des Anspruchs 1 gelöst. Die Erfindung ist nicht auf oder durch dessen Merkmalskombination beschränkt.

Bei allen folgenden Ausführungen ist der Gebrauch des Ausdrucks "kann sein oder haben" synonym zu "ist oder hat vorzugsweise" zu verstehen.

Der erfindungsgemäße Luftabscheider ist zum Abscheiden von Luft aus einem den Luftabscheider durchströmenden Fluid geeignet und weist hierzu wenigstens eine

Luftabscheidekammer auf. Der Luftabscheider oder die Luftabscheidekammer kann eine venöse Blutkammer sein.

Der Begriff "Luftabscheidekammer", wie er hierin verwendet wird, bezeichnet eine Kammer bzw. einen Raum mit einem Kammerinneren, welcher sowohl zum Aufnehmen von Fluiden als auch zum Abscheiden gasförmiger Komponenten aus selbigen geeignet ist. Die Aufnahme kann eine vorübergehende sein.

Der Begriff "Luft" steht im Zusammenhang mit der vorliegenden Erfindung allgemein für "Gas". Die Luftabscheidekammer kann daher auch als eine Gasabscheidekammer verstanden werden.

Der Begriff "Fluid" ist vorzugsweise als "Flüssigkeit" oder Mischungen von Flüssigkeiten zu verstehen ohne hierauf beschränkt zu sein. Auch andere Fluide können erfindungsgemäß unter diesen Begriff fallen. Das Fluid kann gasförmige Anteile aufweisen. Insbesondere solche, welche es im Luftabscheider abzuscheiden gilt.

Allgemein schließt ein Fluid, ohne darauf beschränkt zu sein, Flüssigkeiten, wie medizinische Flüssigkeiten, z. B. Blut, Gase, wie Luft, Emulsionen, Suspensionen, Dispersionen und dergleichen sowie deren Mischungen ein.

Erfindungsgemäß weist die Luftabscheidekammer wenigstens einen Einströmkanal, durch den das Fluid oder der Fluidstrom in die Luftabscheidekammer einströmen kann, und wenigstens einen Abströmkanal, durch welchen das Fluid oder der Fluidstrom aus der Luftabscheidekammer ausströmen kann, auf.

Der Begriff "Einströmkanal", wie er hierin verwendet wird, bezeichnet einen Kanal, eine Leitung oder dergleichen. Der Einströmkanal kann einen geschlossenen oder teilweise geschlossenen Querschnitt oder beides aufweisen. Er kann eine geschlossene oder teilweise offene Struktur oder beides sein.

Gleiches gilt für den Abströmkanal.

Weiterer Merkmale bedarf der erfindungsgemäße Luftabscheider nicht.

Vorteilhafte Weiterentwicklungen der vorliegenden Erfindung sind jeweils Gegenstand des Anspruchs 1, der Unteransprüche und der folgenden Ausführungsformen.

Erfindungsgemäß ist die Luftabscheidekammer in wenigstens eine erste Kammer mit einem ersten Kammerquerschnitt, in welche der Einströmkanal mündet, und wenigstens eine zweite Kammer mit einem zweiten Kammerquerschnitt, aus welcher der Abströmkanal abgeht, unterteilt oder weist derartige erste und zweite Kammern auf.

Der Begriff "Kammerquerschnitt", wie er hierin verwendet wird, bezeichnet insbesondere einen Querschnitt, d. h. eine Abmessung des Umfangs, der ersten Kammer oder der zweiten Kammer in der Richtung der Länge der jeweiligen Kammer.

Der Umfang kann rund oder nicht rund sein. Er kann geschlossen oder offen sein.

Die "Richtung der Länge" einer Kammer kann vorzugsweise eine horizontale Erstreckung während des Gebrauchs des Luftabscheiders oder einer Behandlungsvorrichtung, welche mit einem erfindungsgemäßen Luftabscheider ausgestattet ist, bezeichnen.

Der Kammerquerschnitt kann jedoch auch einen Querschnitt der ersten Kammer oder der zweiten Kammer entlang einer Richtung der Breite der jeweiligen Kammer bezeichnen.

Der Kammerquerschnitt kann ein beliebig gewählter Querschnitt der Kammer sein. Er kann, muss aber nicht, der maximale Kammerquerschnitt der jeweiligen Kammer sein. Er kann ebenso der minimale Kammerquerschnitt sein. Der Kammerquerschnitt kann der Querschnitt an einer bestimmten Position der jeweils ersten Kammer oder zweiten Kammer sein; beispielsweise ein durch einen Mittelpunkt oder Schwerpunkt der jeweiligen Kammer gelegter Querschnitt. Der Kammerquerschnitt kann ferner in einer horizontalen Ebene bestimmt werden, insbesondere in einer Gebrauchsstellung des Luftabscheiders. Der Kammerquerschnitt kann in einer Ebene parallel zu einer Öffnungsfläche oder Haupterstreckungsfläche der Verbindungsöffnung bestimmt werden.

Zwischen der ersten Kammer und der zweiten Kammer ist vorzugsweise wenigstens eine Verbindungsöffnung vorgesehen, durch welche das Fluid aus der ersten Kammer in die zweite Kammer strömen kann. Der Kammerquerschnitt kann senkrecht zu einer Hauptströmungsrichtung des Fluids beim Durchströmen der Verbindungsöffnung sein.

Der Begriff "Verbindungsöffnung", wie er hierin verwendet wird, bezeichnet eine Öffnung oder einen Durchgang bzw. Durchlass für das Fluid.

Durch die Verbindungsöffnung kann die Luftabscheidekammer in die erste Kammer und die zweite Kammer aufgeteilt werden.

Die Verbindungsöffnung kann eine Querschnittsverjüngung aufweisen. Die Verbindungsöffnung (Einschnürung) bewirkt im Wesentlichen eine Entkopplung der Rotationsströmung in der oberen (ersten) Kammer von der im Wesentlichen rotationsfreien Strömung in der unteren (zweiten) Kammer.

Die erste Kammer und die zweite Kammer stehen in unidirektionaler oder in bidirektionaler Fluidkommunikation zueinander. Demnach kann das Fluid oder der Fluidstrom oder ein Teil oder Teilstrom desselben aus der zweiten Kammer ggf. in die erste Kammer einströmen oder zurückströmen.

Das Abscheiden von Luft kann in der ersten Kammer und/oder in der zweiten Kammer der Luftabscheidekammer erfolgen.

Beispielsweise kann die Luft an einer Oberseite der ersten Kammer abgeschieden werden. Die Luft kann zusätzlich oder alternativ in der zweiten Kammer, wie beispielsweise an einer Oberseite oder in einem oberen Bereich oder einer Strömungsnische der zweiten Kammer, abgeschieden werden.

Der Begriff "Oberseite" wie er hierin verwendet wird, sowie alle weiteren Richtungs- oder Anordnungs- bzw. Lagebezeichnungen von Komponenten des erfindungsgemäßen Luftabscheiders, zum Beispiel eine "Unterseite", beziehen sich allgemein bevorzugt auf die Lage bzw. Anordnung des erfindungsgemäßen Luftabscheiders während seiner Verwendung, wie beispielsweise seiner Verwendung in einer Behandlungsvorrichtung.

Der Luftabscheider ist während bzw. zu seiner Verwendung bevorzugt derart angeordnet, dass das Fluid senkrecht - vorzugsweise bezogen auf die Haupterstreckung der Luftabscheidekammer - in den Luftabscheider (nicht zwangsläufig auch in eine Kammer hiervon) einströmt.

Die Erfindung ist hierauf allerdings nicht beschränkt; der Luftabscheider kann während seiner Verwendung alternativ horizontal angeordnet sein.

Die Begriffe "senkrecht" bzw. "vertikal" und "waagrecht" bzw. "horizontal" beziehen sich - wie hier und auch in den Patentansprüchen verwendet - vorzugsweise auf den Erdmittelpunkt.

Der Luftabscheider kann im Gebrauch alternativ in einem beliebigen Winkel bzw. in einer beliebigen Schräge, bezogen auf eine Senkrechte oder eine Horizontale, angeordnet sein. Die Anordnung des Luftabscheiders entspricht während seines Gebrauchs oftmals nicht oder nicht vollkommen der Anordnung bzw. Ausrichtung des Luftabscheiders, wie sie in der beigefügten Zeichnung veranschaulicht ist. Im Folgenden beziehen sich die Richtungsbegriffe ("senkrecht" und/oder "horizontal") der Einfachheit halber dennoch auf die in der Zeichnung dargestellte Anordnung des Luftabscheiders. Etwaige Abweichungen, die sich durch eine Neigung des Luftabscheiders bei dessen Gebrauch ergeben, sind beim Verständnis des Begriffs "senkrecht zum Erdmittelpunkt" entsprechend zu berücksichtigen. Dies gilt auch für die in den Patentansprüchen gewählten Formulierungen.

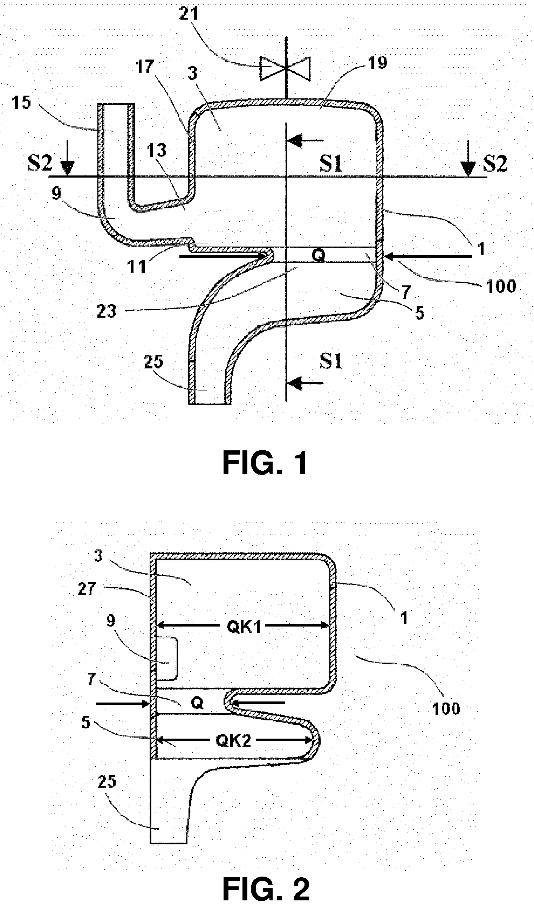

In einer bevorzugten Ausführungsform der vorliegenden Erfindung ist die erste Kammer im Gebrauch des Luftabscheiders oberhalb der zweiten Kammer angeordnet. Eine solche Anordnung ist zum Beispiel in Fig. 1 gezeigt.

Dabei kann die erste Kammer - oder der von ihr umschlossene Raum - vollständig oberhalb der zweiten Kammer - oder deren umschlossenen Raums - liegen. Von der Erfindung ist aber auch umfasst, dass der größere Anteil der ersten Kammer oberhalb des größeren Anteils der zweiten Kammer liegt. Ferner ist umfasst, dass der in die Verbindungsöffnung mündende Anteil der ersten Kammer oberhalb des sich an die Verbindungsöffnung anschließenden Anteils der zweiten Kammer liegt.

In einer weiteren bevorzugten Ausführungsform der vorliegenden Erfindung weist der Luftabscheider wenigstens eine Ventileinrichtung auf, welche zum Entlüften bzw. allgemein Entgasen der Luftabscheidekammer geeignet und/oder angeordnet ist.

Eine "Ventileinrichtung" kann beispielsweise eine Entlüftungsklappe, ein Entlüftungsventil, ein Druckventil, wie ein Überdruckventil, ein Abluftventil, ein Absperrhahn und dergleichen sein. Die Ventileinrichtung kann zum Zwecke der besseren Entlüftung eine Saugeinrichtung wie beispielsweise eine Venturidüse aufweisen.

Mittels der Ventileinrichtung kann die abgeschiedene Luft in ein Äußeres des Luftabscheiders geführt werden. Sie kann beispielsweise in die Atmosphäre abgeführt werden.

Die Ventileinrichtung kann an der Oberseite oder in einem oberen Bereich der ersten Kammer angeordnet sein. Sie kann in einem höchsten Punkt bzw. an einer höchsten Stelle des erfindungsgemäßen Luftabscheiders bzw. der ersten Kammer der Luftabscheidekammer angeordnet sein.

Luft - insbesondere solche, die an der Oberseite bzw. im oberen Bereich der ersten Kammer abgeschieden wurde oder sich dort gesammelt hat- kann mittels der Ventileinrichtung direkt oder indirekt aus der Luftabscheidekammer abgeführt bzw. entfernt werden.

Luft, die in der zweiten Kammer abgeschieden wurde und/oder sich nach Einströmen des Fluids durch die Verbindungsöffnung aus der ersten Kammer in der zweiten Kammer befindet, kann durch die Verbindungsöffnung in die erste Kammer rückgeführt werden. Sie kann ebenfalls durch die oben genannte oder eine andere, ggf. zusätzlich vorgesehene Ventileinrichtung aus der Luftabscheidekammmer abgeführt werden.

Erfindungsgemäß ist der Einströmkanal in einem unteren Bereich des Luftabscheiders oder in einem unteren Bereich der ersten Kammer angeordnet.

Die Anordnung oder Lage des Einströmkanals bzw. der Zuströmung kann so gewählt sein, dass auch bei einem geringen oder gar minimalen Füllstand der ersten Kammer der Zufluss bzw. Zustrom des Fluids unterhalb des Fluidspiegels in der ersten Kammer vorteilhaft gewährleistet sein kann. Dies kann zusätzlich zur Entlüftung oder Entgasung des Fluids beitragen.

Erfindungsgemäß strömt das Fluid tangential zu wenigstens einer Seitenwand der ersten Kammer in diese ein.

Eine "Seitenwand" oder "Wandung" der ersten Kammer bezeichnet eine seitliche Begrenzung der ersten Kammer. Die erste Kammer weist bevorzugt sechs Wände auf, von denen eine im Gebrauch des erfindungsgemäßen Luftabscheiders als Oberseite und
eine weitere als Unterseite zu verstehen ist. Die übrigen Wände werden hier jeweils als "Seitenwand" bezeichnet.

Jede der Seitenwände der ersten Kammer kann im Gebrauch des erfindungsgemäßen Luftabscheiders - unabhängig von der Ausrichtung der übrigen Seitenwände - senkrecht stehen. Sie kann im Wesentlichen senkrecht oder vollständig senkrecht stehen.

Die Wandungen der ersten Kammer können in geeigneter Weise an eine Neigung des erfindungsgemäßen Luftabscheiders, z. B. während seiner Verwendung in einer externen Funktionseinrichtung, angepasst sein. Sie können beispielsweise in geeigneter Weise derart gerundet ausgestaltet sein, dass sie vorteilhaft eine strömungsoptimierte Berührfläche für Fluide darstellen, welche die Luftabscheidekammer durchströmen. Die Optimierung kann darin bestehen, eine geeignete Rotation des einströmenden Fluids zu erreichen. Die Rotation kann sich dadurch auszeichnen, dass sie eine Entlüftung oder eine Entgasung begünstigt und/oder das Vermischen von Fluid und im Inneren der jeweiligen Kammer vorliegender Luft vermeidet oder erschwert.

Der Begriff "tangential" bzw. "tangentiale Einströmung" oder "tangentiale Zuströmung" kann ein Einströmen bzw. Zuströmen des Fluids tangential zu einer Seitenwand der ersten Kammer bezeichnen.

Das Fluid kann seitlich durch den Einströmungskanal (der linken Seite in Fig. 1) in die erste Kammer eingebracht werden. Es kann sich im Weiteren seitlich tangential zu den Seitenwänden der ersten Kammer ausbreiten.

Eine seitlich tangentiale Einströmung kann eine Zone mit im Wesentlichen oder vollständig stabiler Rotationsströmung des Fluids in der Kammer, beispielsweise der ersten Kammer des Luftabscheiders, erzeugen.

Die Ein- bzw. Zuströmung des Fluids kann unter einem Winkel von 0° bis 15°, bezogen auf eine Horizontale, bezogen auf eine Anordnung des Luftabscheiders, wie sie in den beigefügten Fig. dargestellt ist, also beispielsweise leicht nach oben gerichtet, erfolgen. Sie kann jedoch auch nach unten gerichtet erfolgen.

In der vorliegenden Erfindung angegebene Wertebereiche sind hier und im Folgenden allgemein als einschließende Wertebereiche zu verstehen. Die Randbereiche oder -werte sind daher immer als in dem jeweiligen Wertebereich enthalten zu verstehen. Die angegebenen Wertebereiche umfassen ausdrücklich auch alle Zwischenwerte.

In einer weiteren bevorzugten Ausführungsform weitet sich ein Längs- oder ein Querschnitt des Einströmkanals in Richtung zur Luftabscheidekammer und insbesondere zur ersten Kammer hin auf.

Eine beispielhafte Anordnung ist in der beigefügten Fig. 1 gezeigt.

Der Längs- oder Querschnitt des Einströmkanals kann sich kontinuierlich oder stetig aufweiten. Der Längs- oder Querschnitt kann sich jedoch auch abschnittsweise bzw. schrittweise, d. h. beispielsweise in diskreten Stufen, aufweiten.

In einer weiteren bevorzugten Ausführungsform der vorliegenden Erfindung kann ein Winkel der Aufweitung des Längsschnitts des Einströmkanals, bezogen auf einen Längsschnitt eines nicht aufgeweiteten Abschnitts des Einströmkanals, auf wenigstens einer Längswand (z. B. oben oder unten) des Einströmkanals zwischen 0° und (einschließlich) 15° betragen. Die Aufweitung bzw. der aufgeweitete Abschnitt des Einströmkanals kann die Form eines Diffusors aufweisen.

In einer weiteren bevorzugten Ausführungsform ist ein Strömungsquerschnitt oder ein durchströmter Querschnitt der Verbindungsöffnung kleiner als der Kammerquerschnitt der ersten Kammer und/oder der zweiten Kammer.

Der Begriff "Strömungsquerschnitt", wie er hierin verwendet wird, bezeichnet einen Querschnitt der Verbindungsöffnung, welcher allgemein senkrecht zur Hauptströmungsrichtung des Fluids verläuft.

Bei dem Strömungsquerschnitt der Verbindungsöffnung kann es sich, analog der vorstehend angegebenen Definition eines Kammerquerschnitts, um einen maximalen Querschnitt oder einen minimalen Querschnitt oder einen beliebigen anderen Querschnitt der Verbindungsöffnung senkrecht zur Richtung des Fluidstroms handeln.

Der Begriff "kleiner", wie er hierin verwendet wird, meint, dass der Strömungsquerschnitt der Verbindungsöffnung im Wesentlichen kleiner als der Kammerquerschnitt der ersten Kammer und/oder der zweiten Kammer ist, und zwar in wenigstens einer Dimension (Tiefe, Breite, usw.) und/oder bezogen auf eine Querschnittsfläche. Der Begriff "Querschnitt" sowie seine Zusammensetzung kann sowohl eine Fläche, als auch eine Entfernung bzw. Distanz bezeichnen.

Der Strömungsquerschnitt kann beispielsweise halb so groß wie ein Kammerquerschnitt sein.

In einer weiteren bevorzugten Ausführungsform der vorliegenden Erfindung ist die Verbindungsöffnung in Bezug auf eine während der Verwendung des Luftabscheiders senkrechte Mittelachse der ersten Kammer und/oder der zweiten Kammer außermittig angeordnet.

Der Begriff "senkrechte Mittelachse" bezieht sich hier und im Folgenden auf eine "umgekippte" Anordnung der externen Funktionseinrichtung, wie sie in den beigefügten Fig. dargestellt ist. Wenn die externe Funktionseinrichtung gekippt bzw. geneigt (z. B. bis 15°) montiert wird (z. B. an einer Behandlungsvorrichtung), trifft dies hingegen nicht mehr zu.

Eine derartige "außermittige" Anordnung kann beispielsweise bedeuten, dass Mittelachsen des Luftabscheiders und der Verbindungsöffnung und/oder Mittelachsen der ersten Kammer und der Verbindungsöffnung und/oder Mittelachsen der zweiten Kammer und der Verbindungsöffnung nicht zusammenfallen.

Eine außermittige Anordnung der Verbindungsöffnung kann auch derart definiert sein, dass die Mittelachse der Verbindungsöffnung zu beispielsweise einer Rotationsachse einer rotationssymmetrischen ersten Kammer und/oder zweiten Kammer und/oder eines rotationssymmetrischen Luftabscheiders versetzt angeordnet ist.

Wenn der Strömungsquerschnitt der Verbindungsöffnung zwischen der ersten Kammer und der zweiten Kammer kleiner als der Kammerquerschnitt der zweiten Kammer ist, kann sich die Strömung bzw. Strömungsgeschwindigkeit des aus der ersten Kammer durch die Verbindungsöffnung in die zweite Kammer einströmenden Fluids verlangsamen.

Die zweite Kammer kann beispielsweise eine Beruhigungszone für die Fluidströmung darstellen. Es ist möglich, dass in einer derartigen Beruhigungszone im Wesentlichen keine oder überhaupt keine Rotationsströmung des Fluids vorliegt.

Ein derartiges verlangsamtes Einströmen des Fluids aus der ersten Kammer, durch die Verbindungsöffnung in die zweite Kammer kann vorteilhaft dazu beitragen, Luftblasen in der zweiten Kammer abzuscheiden.

Beispielsweise können Luftblasen, die in der ersten Kammer nicht aus dem Fluid oder Fluidstrom abgeschieden wurden, in der zweiten Kammer abgeschieden werden.

Die Blasen können sich zum Beispiel an einer Oberseite bzw. in einem oberen Bereich der zweiten Kammer sammeln.

Die Blasen können durch die Verbindungsöffnung in die erste Kammer zurückströmen. Die Blasen können bei einer senkrechten Ausrichtung des Luftabscheiders während seines Gebrauchs aus der zweiten Kammer in die erste Kammer aufsteigen.

Derartige, in die erste Kammer zurückgeströmte oder aufgestiegene Luft kann sich an der Oberseite bzw. in einem oberen Bereich der ersten Kammer sammeln. Sie kann durch die Ventileinrichtung aus dem Luftabscheider ausgetragen werden.

Der erfindungsgemäße Luftabscheider kann ein Gehäuse aufweisen.

Beispielsweise kann die Luftabscheidekammer als Gehäuse ausgestaltet sein. Die Luftabscheidekammer kann aus einem Gehäuse gebildet sein, welches die erste Kammer und die zweite Kammer aufweist.

Unter dem Begriff "Gehäuse", wie er hierin verwendet wird, ist ein Grundkörper zu verstehen, welcher insbesondere durch die Oberseite, die Unterseite und wenigstens zwei Seitenwänden der Luftabscheidekammer gebildet wird.

Zum Beispiel kann das Gehäuse des Luftabscheiders ein in eine erste Kammer und eine zweite Kammer unterteilten Grundkörper aufweisen.

In einer bevorzugten Ausführungsform der vorliegenden Erfindung weist der Luftabscheider eine Abdeckungseinrichtung auf.

Der Begriff "Abdeckungseinrichtung", wie er hierin verwendet wird, bezeichnet eine Einrichtung, welche ein Inneres bzw. einen Innenraum des Grundkörpers, d. h. der Luftabscheidekammer oder des Luftabscheiders, abdeckt bzw. bedeckt.

Die Abdeckungseinrichtung kann das Innere bzw. den Innenraum des Luftabscheiders oder der Luftabscheidekammer gegen ein Äußeres verschließen und/oder abdichten. Die Abdeckungseinrichtung kann das Innere des Luftabscheiders fluiddicht gegen ein Äußeres abdichten. Gegebenenfalls kann die Abdeckungseinrichtung das Innere des Luftabscheiders hermetisch gegen ein Äußeres abdichten.

Unter dem Begriff "Äußeres des Luftabscheiders" kann die Atmosphäre verstanden werden. Es kann ebenso jeder Raum außerhalb des Luftabscheiders verstanden werden, beispielsweise ein Inneres bzw. Innenraum einer Behandlungsvorrichtung oder einer externen Funktionseinrichtung wie einer Blutkassette, in welcher der erfindungsgemäße Luftabscheider eingesetzt wird.

Die Abdeckungseinrichtung kann in bzw. an wenigstens einem Abschnitt einer Seitenwand der Luftabscheidekammer vorgesehen sein. Die Abdeckungseinrichtung kann eine Seitenwand der Luftabscheidekammer bilden.

Die Abdeckungseinrichtung kann als separate Komponente ausgestaltet sein. Sie kann am Grundkörper befestigt sein.

Die Abdeckungseinrichtung kann ein Deckel sein.

Ein "Deckel" kann eine formfeste und/oder relativ steife Abdeckungseinrichtung sein. Der Deckel kann z. B. an einer Seite desselben mit bzw. an der Luftabscheidekammer befestigt sein. Der Deckel kann durch Öffnen bzw. Abheben desselben von der Seitenwand der Luftabscheidekammer oder dem Grundkörper der Luftabscheidekammer einen Zugang zum Inneren der Luftabscheidekammer gewähren.

Der Deckel kann vorzugsweise flach, d. h. zum Beispiel im Wesentlichen eben, ausgestaltet sein. Der Deckel kann gewölbt oder wellig ausgestaltet sein.

Die Abdeckungseinrichtung kann eine Folie sein.

Eine "Folie" kann, z. B. im Gegensatz zu einem Deckel, eine flexible oder biegsame und/oder nachgiebige Abdeckungseinrichtung sein.

Die Folie kann einen Zugang oder Zugriff auf das bzw. zum Inneren des Luftabscheiders durch die Folie hindurch zulassen.

In einer bevorzugten Ausführungsform der vorliegenden Erfindung, in welcher eine Folie als Abdeckungseinrichtung an einer Seitenwand der Luftabscheidekammer vorgesehen ist, kann die Ventileinrichtung zum Entlüften beispielsweise als Phantomventil ausgestaltet sein.

Der Begriff "Phantomventil", wie er hierin verwendet wird, bezeichnet ein Element mit einer mittels Aktor erreichbaren Aktor-Fläche (hier beispielsweise eine Aktor-Membran), welches die Funktion eines Ventils übernehmen kann.

Die Aktor-Membran ist unter Aufbringung einer Kraft hierauf, z.B. einer Druckkraft, in eine Richtung bewegbar, ausdehnbar bzw. wölbbar oder dergleichen. Durch das Bewegen oder Ausdehnen der Aktor-Membran kann sich diese an ein Element, wie eine Abdichtungseinrichtung, etwa einen Steg, anlegen oder von diesem entfernen. Die Aktor-Membran kann somit beispielsweise eine Abdichtung bewirken bzw. verstärken oder beenden bzw. verringern.

Wenn die Kraft auf die Aktor-Membran aufgehoben wird, kann diese in beispielsweise eine Grundposition, z. B. einen nicht gewölbten Zustand, zurückkehren.

Ein derartiges Phantomventil ist beispielsweise in der von der Anmelderin der vorliegenden Erfindung beim DPMA am 10. März 2009 hinterlegten Patentanmeldung 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren"* beschrieben.

In einer weiteren bevorzugten Ausführungsform der vorliegenden Erfindung kann der Luftabscheider wenigstens eine Erfassungseinrichtung zum Erfassen eines Zustands und/oder einer Änderung eines Zustands eines Kammerinneren des Luftabscheiders aufweisen.

Der Begriff "Erfassungseinrichtung", wie er hierin verwendet wird, bezeichnet eine zum Erfassen bzw. Detektieren und/oder Messen, Aufnehmen, Aufzeichnen und dergleichen von Werten oder Messdaten geeignete Einrichtung.

Eine derartige Erfassungseinrichtung kann ein Sensor, eine Sonde, ein Messfühler und dergleichen sein.

Das Erfassen eines Zustands und/oder einer Änderung eines Zustands eines Kammerinneren des Luftabscheiders kann beispielsweise durch Messen wenigstens eines Parameters bzw. wenigstens einer Zustandsgröße, die zum Beschreiben des Zustands und/oder einer Änderung des Zustands des Kammerinneren geeignet ist, erfolgen.

Beispielsweise kann die Erfassungseinrichtung zum Erfassen bzw. Messen eines Füllstands der Luftabscheidekammer, eines Drucks im Inneren der Luftabscheidekammer, einer Temperatur im Inneren der Luftabscheidekammer und dergleichen eingesetzt werden.

Wenn die Abdeckungseinrichtung eine Folie ist, kann das Erfassen bzw. Messen des Zustands oder einer Änderung des Zustands des Kammerinneren des Luftabscheiders beispielsweise durch die Abdeckungseinrichtung hindurch erfolgen. Dies kann vorteilhaft ein nicht-invasives Messen oder Erfassen eines Zustands oder der Änderung eines Zustands im Inneren des erfindungsgemäßen Luftabscheiders sein.

Zu diesem Zweck kann die Folie eine geeignete Wandstärke, insbesondere eine ausreichend dünne Wandstärke, aufweisen.

In einer weiteren bevorzugten Ausführungsform ist der Abströmkanal derart ausgestaltet, dass unterhalb des Abströmkanals befindliche Luft bei Strömungsstillstand über die zweite, bspw. die untere, Kammer und die erste, bspw. die obere, Kammer des Luftabscheiders abführbar ist.

Das Abführen von Luft bzw. Luftblasen aus einem Abschnitt oder Bereich des Luftabscheiders aus einem unteren Bereich der zweiten Kammer oder einem Bereich unterhalb der zweiten Kammer kann durch geeignete Anordnung und/oder Ausgestaltung des Abströmkanals begünstigt werden.

Beispielsweise kann der Längs- oder Querschnitt des Abströmkanals und/oder eine Krümmung desselben geeignet gewählt werden.

Ein Beispiel einer bevorzugten Anordnung bzw. Ausgestaltung des Abströmkanals ist in Fig. 1 gezeigt.

Der erfindungsgemäße Luftabscheider kann als Einzelteil gefertigt sein. Er kann als Teil einer externen Funktionseinrichtung gefertigt sein, wahlweise zum Einsetzen oder als integrales oder einstückiges Bauteil. Beispielsweise kann der Luftabscheider mittels Spritzguss herstellt sein.

Die erfindungsgemäße Aufgabe wird gleichermaßen durch eine erfindungsgemäße externe Funktionseinrichtung gemäß Anspruch 11 gelöst. Alle Vorteile des erfindungsgemäßen Luftabscheiders lassen sich ungeschmälert auch mit der erfindungsgemäßen externen Funktionseinrichtung erreichen.

Eine erfindungsgemäße externe Funktionseinrichtung weist wenigstens einen erfindungsgemäßen Luftabscheider auf.

Eine "externe Funktionseinrichtung" kann eine Einmalkomponente oder ein Einmalartikel sein. Sie kann aus einem Kunststoffmaterial gefertigt sein.

Die externe Funktionseinrichtung bzw. ein Hartteil derselben kann mittels Spritzgießen, Spritzblasen, Tiefziehen und dergleichen hergestellt werden.

Die erfindungsgemäße externe Funktionseinrichtung kann zur Verwendung in einem Behandlungsverfahren vorgesehen sein. Behandlungsverfahren im Sinne der vorliegenden Erfindung schließen medizinische oder medizintechnische Behandlungsverfahren sowie Behandlungsverfahren der Labortechnik ein.

In einer bevorzugten Ausführungsform ist die erfindungsgemäße externe Funktionseinrichtung als Blutkassette ausgestaltet.

Eine Blutkassette im Sinne der vorliegenden Erfindung ist beispielsweise in der von der Anmelderin der vorliegenden Erfindung in der beim DPMA am 23. April 2009 eingereichten Anmeldung 10 2009 018 664.6 bzw. in der am 10. Juni 2009 eingereichten Anmeldung 10 2009 024 468.9 mit jeweils dem Titel *"Externe Funktionseinrichtung, Blutbehandlungsvorrichtung zum Aufnehmen einer erfindungsgemäßen externen Funktionseinrichtung, sowie Verfahren"* beschrieben.

In einer weiteren bevorzugten Ausführungsform ist die externe Funktionseinrichtung als Spritzguss-Hartteil mit einer aufgeschweißten Folie ausgestaltet.

Die erfindungsgemäße Aufgabe wird gleichermaßen durch ein erfindungsgemäßes Schlauchsystem zum extrakorporalen Führen von Blut (hierin auch als Blutkreislauf bezeichnet) gemäß Anspruch 13 gelöst. Alle Vorteile des erfindungsgemäßen Luftabscheiders lassen sich ungeschmälert auch mit dem erfindungsgemäßen Schlauchsystem erreichen.

Ein erfindungsgemäßer Blutkreislauf weist wenigstens einen erfindungsgemäßen Luftabscheider auf und/oder ist mit wenigstens einer erfindungsgemäßen externen Funktionseinrichtung verbunden.

Der Begriff "Blutkreislauf" wie er hierin verwendet wird, kann als ein Schlauchsystem extrakorporal Blut führen.

Sowohl die externe Funktionseinrichtung als auch der Blutkreislauf können zur Verwendung in oder an einer Behandlungsvorrichtung vorgesehen sein.

Die erfindungsgemäße Aufgabe wird auch durch eine erfindungsgemäße Behandlungsvorrichtung gemäß Anspruch 14 gelöst. Alle Vorteile des erfindungsgemäßen Luftabscheiders lassen sich wiederum ungeschmälert mit der erfindungsgemäßen Behandlungsvorrichtung erreichen.

Eine erfindungsgemäße Behandlungsvorrichtung weist wenigstens einen erfindungsgemäßen Luftabscheider und/oder wenigstens eine erfindungsgemäße externe Funktionseinrichtung und/oder wenigstens ein erfindungsgemäßes Schlauchsystem auf.

In einer bevorzugten Ausführungsform der vorliegenden Erfindung darf der Luftabscheider oder die externe Funktionseinrichtung während seines/ihres Gebrauchs gegen eine Vertikale geneigt sein. Auch bei einer derartigen Neigung ist es vorteilhaft möglich, die aus dem Fluid ausgeschiedene Luft in einen zur Entlüftung geeigneten Bereich, z. B. im Bereich einer Ventileinrichtung an einer Oberseite der entsprechenden Kammer anzusammeln. Dies begünstigt die Entlüftung und kann zu einer robusteren Arbeitsweise des Luftabscheiders beitragen.

Ein derartiger "Neigungswinkel" oder Winkel der Neigung kann zum Beispiel zwischen -15° und +15° betragen, wobei die Zwischen- und Randwerte jeweils mit eingeschlossen sind.

In einer bevorzugten Ausführungsform der vorliegenden Erfindung ist die Behandlungsvorrichtung eine Blutbehandlungsvorrichtung, wie eine Dialysiervorrichtung zum Durchführen einer Dialysebehandlung, wie einer Hämodialyse, Hämofiltration, Hämodiafiltration und dergleichen.

Die vorliegende Erfindung ist hier beschrieben als eine Vorrichtung zum Abscheiden von Luft aus extrakorporal strömendem Blut. Die vorliegende Erfindung soll jedoch ausdrücklich nicht hierauf beschränkt sein. Ein Entfernen eines beliebiges Gases aus einem beliebigen Fluid, insbesondere aus einer Flüssigkeit, fällt ebenfalls unter die hier dargelegte Erfindung. Daher ist die Verwendung der vorliegenden Erfindung nicht auf einen medizinischen Einsatz und auf die Verwendung bei extrakorporalen Blutkreisläufen beschränkt.

Der vorliegende Luftabscheider ist zum Abscheiden bzw. Entfernen von Luft oder anderen Gasen aus einem den Luftabscheider durchströmenden Fluid geeignet. So kann er beispielsweise vorteilhaft in einem extrakorporalen Blutkreislauf dazu eingesetzt werden, Luftblasen abzuscheiden, welche im extrakorporal strömenden Blut mitströmen bzw. mitschwimmen. Auf diese Weise kann es vorteilhaft möglich sein, das Infundieren von Luftblasen bei der Blutrückgabe an den Patienten in den Blutkreislauf des Patienten zu verhindern.

Die erfindungsgemäße Luftabscheidekammer kann dazu vorgesehen sein, eine Mindestmenge der abgeschiedenen Luft, zum Beispiel in einem oberen Bereich der ersten Kammer, zu speichern. Mit Hilfe der Ventileinrichtung kann die Luft, beispielsweise während des Füllens der Luftabscheidekammer mit Fluid und/oder aus der bereits mit Fluid gefüllten Luftabscheidekammer, vorteilhaft jederzeit im Wesentlichen vollständig entfernt werden. Auf diese Weise kann vorteilhaft ein störungsfreier und/oder im Wesentlichen vollständig luftfreier Betrieb des extrakorporalen Blutkreislaufs gewährleistet werden. Die Gefahr die in einem extrakorporalen Blutkreislauf befindliche Luft während der Blutrückgabe in einen Patienten zu infundieren, kann somit vorteilhaft verringert oder gar vermieden werden.

Die Abscheidung von Luft aus einem Fluidstrom kann mit Hilfe des erfindungsgemäßen Luftabscheiders vorteilhaft optimiert werden. Dies kann zum einen durch die Aufteilung des Luftabscheiders in eine erste Kammer - mit einer Zone mit im Wesentlichen oder vollständig stabiler Rotationsströmung des Fluids - und in eine zweite Kammer - mit einer Beruhigungszone für die Fluidströmung - begünstigt werden.

Die Rotationsströmung des Fluids in der ersten Kammer kann beispielsweise durch tangentiale Anströmung oder Zuströmung des Fluids zu einer Seitenwand oder Seitenwänden der Luftabscheidekammer hin erreicht werden. Auf diese Weise kann die Luft bereits vorteilhaft während des Einströmens des Fluids in die erste Kammer abgeschieden werden.

Die Abscheidung der Luft an der Oberseite der ersten Kammer kann vorteilhaft durch Anordnen des Einströmkanals in einem unteren Bereich der ersten Kammer begünstigt werden. So kann sich die Luft vorteilhaft bereits während des Einströmens im oberen Bereich der ersten Kammer sammeln und direkt durch die Ventileinrichtung aus der Kammer entfernt werden.

Die Abscheidung der Luft an der Oberseite der ersten Kammer kann durch Aufweiten des Einströmkanals zur Luftabscheidekammer hin weiter optimiert werden. Durch die Aufweitung kann die Einströmgeschwindigkeit des Fluids vorteilhaft derart verlangsamt werden, dass Luftblasen aus dem einströmenden Fluid in den oberen Bereich der Kammer aufsteigen und sich dort sammeln können.

U. a. durch außermittiges Anordnen der Verbindungsöffnung zwischen der ersten Kammer und der zweiten Kammer kann vorteilhaft erreicht werden, dass ein Wirbelkern der sich in der ersten Kammer ausbildenden Rotationsströmung des einströmenden Fluids in einem unteren Bereich und/oder an der unteren Wand oder Unterseite der ersten Kammer endet. Auf diese Weise kann der Wirbel bzw. die Wirbel- oder Rotationsströmung des Fluids in der ersten Kammer vorteilhaft stabilisiert und ein Abströmen der Luftblasen aus dem Wirbelkern nach unten, d. h. in die zweite Kammer, verringert oder sogar ganz vermieden werden.

Durch die erfindungsgemäße Ausgestaltung des Strömungsquerschnitts der Verbindungsöffnung und/oder Ausgestaltung und/oder Anordnung des Abströmkanals kann es ferner vorteilhaft möglich sein, Luft, die nicht bereits während des Einströmens des Fluids abgeschieden wurde, aus der zweiten Kammer und/oder von unterhalb der zweiten Kammer zu entfernen.

Mit dem erfindungsgemäßen Luftabscheider kann somit vorteilhaft eine effiziente Abscheidung für Luft aus dem Blut oder Fluid erzielt werden.

Der Grundkörper des erfindungsgemäßen Luftabscheiders kann als Teil einer externen Funktionseinrichtung, wie einer Blutkassette, so ausgestaltet sein, dass er auch bei geneigtem Einbau in beispielsweise einer Blutbehandlungsvorrichtung ein Sammeln der Luft in einem oberen Bereich der entsprechenden, beispielsweise ersten Kammer, insbesondere im Bereich einer Ventileinrichtung, gewährleisten kann. Auf diese Weise kann das im Wesentlichen vollständige Entfernen von Blut auch aus einem während seiner Verwendung mit einer Neigung angeordneten Luftabscheider gewährleistet sein.

Im Folgenden wird die vorliegende Erfindung unter Bezugnahme auf die beigefügte Zeichnung beschrieben. In der Zeichnung bezeichnen identische Bezugszeichen gleiche oder identische Elemente. Es zeigt:
- Fig. 1: eine Frontansicht bzw. Ansicht von vorne auf den erfindungsgemäßen Luftabscheider gemäß einer ersten Ausführungsform,
- Fig. 2: einen Längsschnitt durch einen Luftabscheider gemäß der ersten Ausführungsform;
- Fig. 3: einen Querschnitt des Luftabscheiders aus Fig. 2 mit einer flachen Abdeckungseinrichtung; und
- Fig. 4: einen möglichen Querschnitt des erfindungsgemäßen Luftabscheiders mit einer gewölbten Abdeckungseinrichtung;
- Fig. 5: eine Seitenansicht einer an ihrer Vorderseite mit einer Abdeckungseinrichtung versehenen erfindungsgemäßen Funktionseinrichtung gemäß einer bevorzugten Ausführungsform;
- Fig. 6: die externe Funktionseinrichtung der Fig. 5 mit nach zerstörendem Aufschneiden aufgeklappter Abdeckungseinrichtung;
- Fig. 7: die externe Funktionseinrichtung aus Fig. 5 und Fig. 6, betrachtet von ihrer Rückseite; und
- Fig. 8: schematisch vereinfacht eine weitere Ausführungsform des erfindungsgemäßen Luftabscheiders.

**Fig. 1** zeigt eine Vorderansicht eines Luftabscheiders gemäß der vorliegenden Erfindung.

Der Luftabscheider 100 weist eine Luftabscheidekammer 1 auf, die in eine erste Kammer 3 und eine zweite Kammer 5 unterteilt ist. Der Luftabscheider 100 ist hier exemplarisch als eine venöse Blutkammer ausgestaltet.

In Fig. 1 ist die erste Kammer 3 oberhalb der zweiten Kammer 5 angeordnet. Diese Anordnung kann der üblichen Verwendung des erfindungsgemäßen Luftabscheiders entsprechen. Die erste Kammer 3 kann bei einer solchen Anordnung daher gleichbedeutend als obere Kammer bezeichnet werden, die zweite Kammer 5 als untere Kammer.

Zwischen der ersten Kammer 3 und der zweiten Kammer 5 ist eine Verbindungsöffnung 7 angeordnet. Die Verbindungsöffnung 7 weist einen geringeren Strömungsquerschnitt Q als die zweite Kammer 5 auf.

Ein Einströmkanal 9 steht in Fluidkommunikation mit der ersten Kammer 3 und ermöglicht das Einströmen eines Fluids in die erste Kammer 3.

Wie in Fig. 1 gezeigt, ist der Einströmkanal 9 in einem unteren Bereich 11 der ersten Kammer 3 vorgesehen.

Der Einströmkanal 9 weist eine Aufweitung 13 auf. Ein Längsschnitt der Aufweitung 13 hat breitere Abschnitte als ein Längsschnitt des Einströmkanals 9 in einem nicht aufgeweiteten Bereich 15.

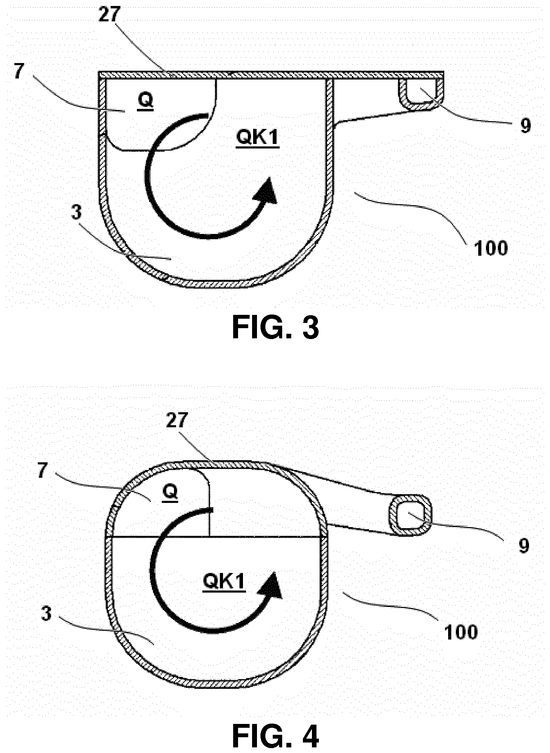

Während des Gebrauchs strömt Fluid durch den Einströmkanal 9 in die erste Kammer 3 ein. Bei einer Anordnung, wie sie beispielhaft in Fig. 1 gezeigt ist, erfolgt eine Ein- bzw. Zuströmung des Fluids in die erste Kammer 3 tangential zu einer Seitenwand 17, wie dies insbesondere auch aus den Fig. 3 und 4 ersichtlich ist.

Das Fluid strömt in Richtung zu einer Oberseite bzw. einen oberen Bereich 19 der ersten Kammer 3 hin.

Im Fluid enthaltene Luft wird in der ersten Kammer 3, insbesondere im oberen Bereich 19 der ersten Kammer 3, abgeschieden und kann durch eine Ventileinrichtung 21 aus der ersten Kammer 3 entfernt werden.

Das Fluid strömt aus der ersten Kammer 3 durch die Verbindungsöffnung 7 in die zweite Kammer 5. Aufgrund der Querschnittsverjüngung der Verbindungsöffnung 7 in Bezug auf einen Kammerquerschnitt der zweiten Kammer 5 - oder des kleineren Querschnitts, einer kleineren Dimension oder einer kleineren Fläche der Durchtrittsöffnung jeweils gegenüber der zweiten Kammer 5 - verlangsamt sich die Strömungsgeschwindigkeit des Fluids nach dem Eintritt in die zweite Kammer 5.

Luft, welche noch nicht während des Einströmens des Fluids in die erste Kammer 3 abgeschieden wurde, kann in einem oberen Bereich 23 der zweiten Kammer 5 abgeschieden werden. Sie kann durch die erste Kammer 3 in den oberen Bereich 19 der ersten Kammer 3 geführt werden bzw. in diesen aufsteigen und mit Hilfe der Ventileinrichtung 21 aus der ersten Kammer 3 entfernt werden.

Die zweite Kammer 5 ist mit einem Abströmkanal 25 verbunden.

Der Abströmkanal 25 ist vorzugsweise so ausgelegt, dass Luft, welche noch nicht in der ersten Kammer 3 oder in der zweiten Kammer 5 abgeschieden wurde, bei Stillstand der Strömung durch den Abströmkanal 25 in die zweite Kammer 5 und von dort durch die Verbindungsöffnung 7 in die erste Kammer 3 bzw. in einen oberen Bereich 19 derselben abgeführt wird. Sie kann von dort aus der ersten Kammer 3 entfernt werden.

**Fig. 2** zeigt eine Seitenansicht des erfindungsgemäßen Luftabscheiders 100 in Form eines Längsschnitts durch den Luftabscheider aus Fig. 1 entlang der dort markierten Linie S1-S1.

In der Darstellung in Fig. 2 ist gut zu erkennen, dass die Verbindungsöffnung 7 außermittig zwischen der ersten Kammer 3 und der zweiten Kammer 5 angeordnet ist. Sie hat ferner einen kleineren Strömungsquerschnitt Q gemessen an einem Strömungsquerschnitt QK1 der ersten Kammer 3. Sie hat zudem einen kleineren Strömungsquerschnitt gemessen an einem Strömungsquerschnitt QK2 der zweiten Kammer.

Der Luftabscheider 100 weist eine Abdeckungseinrichtung 27 an einer Seite (der linken Seite in Fig. 2) auf.

**Fig. 3** zeigt einen Querschnitt des Luftabscheiders 100 gemäß der vorliegenden Erfindung entlang einer Horizontalen des Luftabscheiders 100, die in Fig. 1 als Linie S2-S2 gekennzeichnet ist.

Dieser Querschnitt zeigt einen Blick auf die erste Kammer 3, wobei der Betrachter des in Fig. 3 Gezeigten von oben auf den Luftabscheider 100, also auf eine Oberseite desselben, blickt.

Die tangentiale Zu- bzw. Einströmung des Fluids durch den Einströmkanal 9 in die erste Kammer 3 ist durch den gegen den Uhrzeigersinn in Fig. 3 teilkreisförmig verlaufenden Pfeil angedeutet.

Die Abdeckungseinrichtung 27 ist flach ausgestaltet.

**Fig. 4** zeigt ebenfalls einen Querschnitt eines erfindungsgemäßen Luftabscheiders 100.

Die Ausführungen der Fig. 3 und Fig. 4 entsprechen sich im Wesentlichen, außer, dass die Abdeckungseinrichtung 27 in Fig. 4 gewölbt ausgeführt ist.

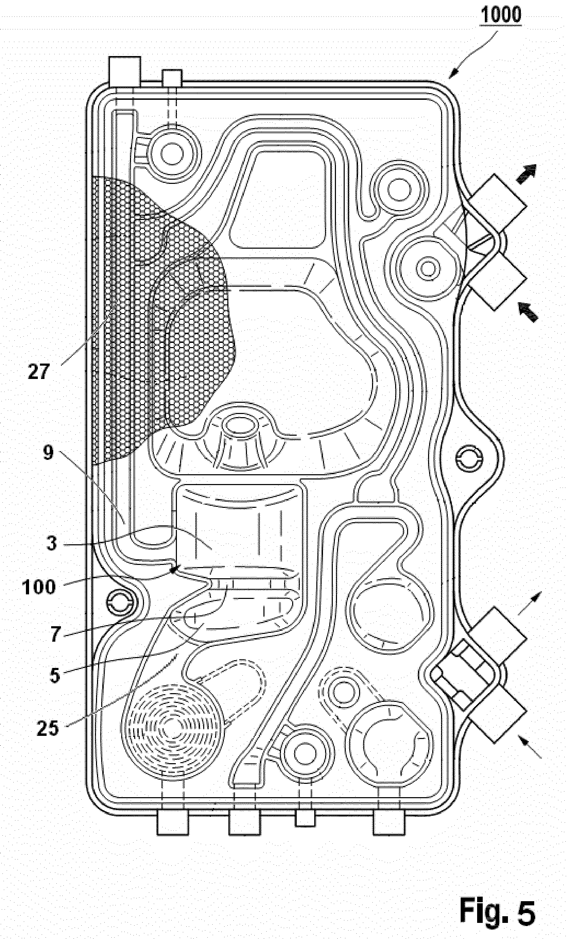

**Fig. 5** zeigt eine Seitenansicht einer erfindungsgemäßen externen Funktionseinrichtung, welche an der Oberfläche, auf die in Fig. 5 geblickt wird, mit einer Abdeckungseinrichtung 27 versehen ist.

Die externe Funktionseinrichtung ist hier exemplarisch als Blutbehandlungskassette 1000 ausgestaltet mit Kammern, Kanälen, Ventilen und dergleichen. Die Abdeckungseinrichtung 27 deckt eine Vorderseite der Blutbehandlungskassette 1000 ab. Sie ist beispielhaft als Folie ausgestaltet.

Die Blutbehandlungskassette 1000 kann wenigstens mit der in Fig. 5 gezeigten Vorderseite an eine Blutbehandlungsvorrichtung (in Fig. 5 nicht gezeigt) angekoppelt werden.

Die Blutbehandlungskassette 1000 weist einen erfindungsgemäßen Luftabscheider 100 auf.

Der Luftabscheider 100 weist die erste Kammer 3 und die zweite Kammer 5 auf, welche durch die Verbindungsöffnung 7 voneinander getrennt sind.

Die erste Kammer 3 ist mit dem Einströmkanal 9 verbunden, die zweite Kammer 5 mit dem Abströmkanal 25.

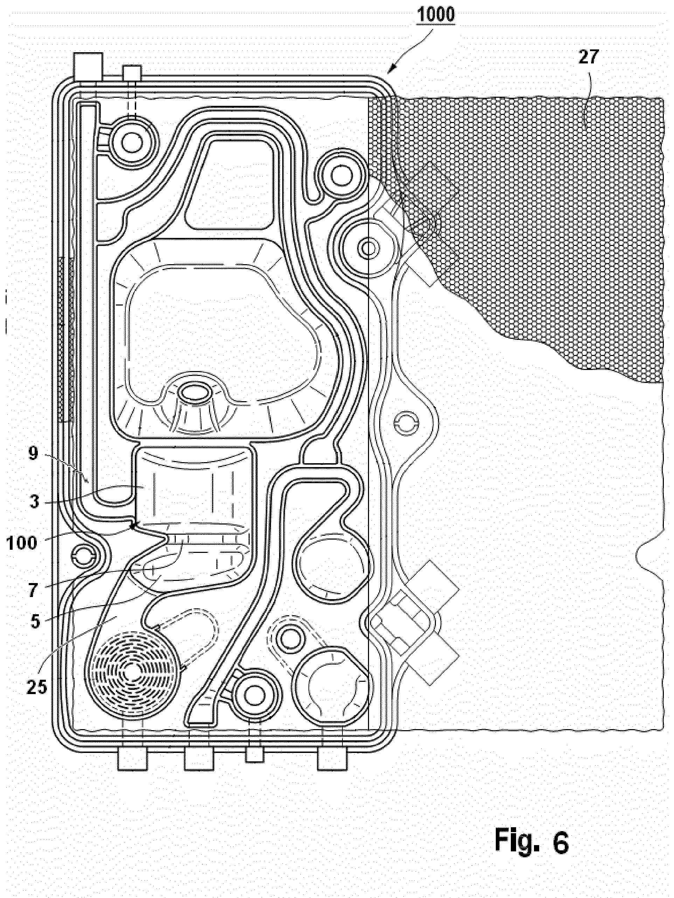

**Fig. 6** zeigt die Blutbehandlungskassette 1000 der Fig. 5, wobei die Abdeckungseinrichtung 27 am linken Rand der Blutbehandlungskassette 1000 sowie oben und unten zerstörend aufgeschnitten und nach rechts aufgeklappt zu erkennen ist.

Fig. 6 zeigt die nach Aufschneiden der Folie detaillierter zu erkennenden Elemente im Inneren der Blutbehandlungskassette 1000.

Zur Vermeidung von Wiederholungen wird auf die vorstehend bei der Beschreibung der Fig. 5 erörterten Ausgestaltungen der einzelnen Elemente verwiesen.

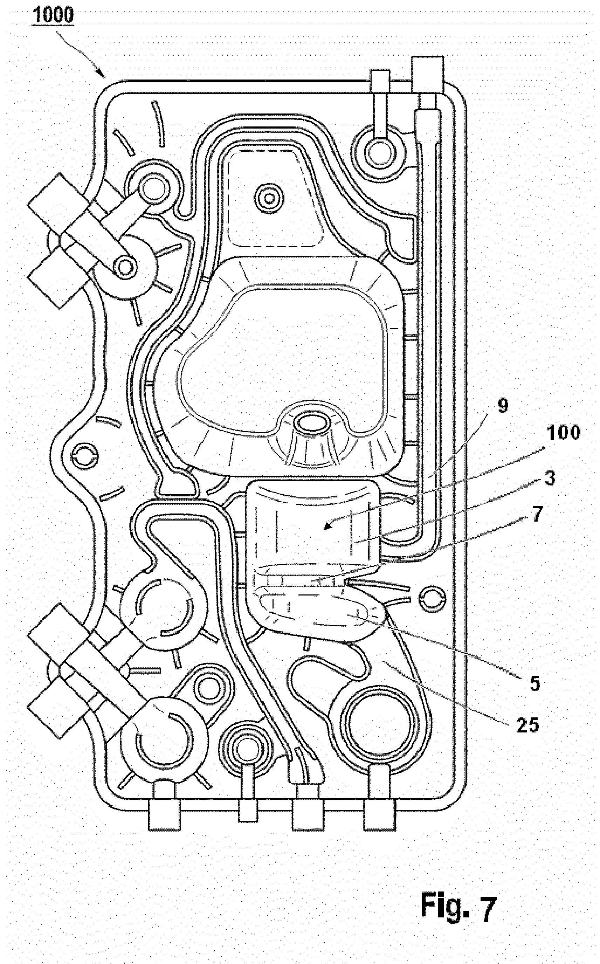

**Fig. 7** zeigt die Blutbehandlungskassette 1000 von ihrer Rückseite. Ist die Blutbehandlungskassette 1000 an die Blutbehandlungsvorrichtung angekoppelt, so wird ein Betrachter beim Öffnen einer Tür der Blutbehandlungsvorrichtung zum Entnehmen der Blutbehandlungskassette 1000 auf diese Rückseite blicken.

Für weitere Details zur Blutbehandlungskassette 1000 wird auf deren ausführliche Beschreibung in den hierauf gerichteten, o.g. Anmeldungen verwiesen. Deren Inhalt ist hiermit in die vorliegende Beschreibung aufgenommen.

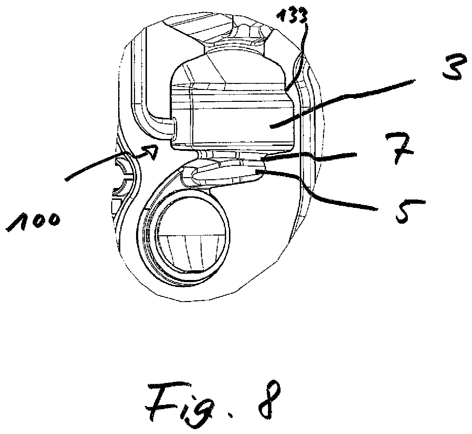

**Fig. 8** zeigt schematisch vereinfacht eine weitere Ausführungsform des erfindungsgemäßen Luftabscheiders 100.

Wie in Fig. 8 zu erkennen ist, weist der erfindungsgemäße Luftabscheider 100 eine Einbuchtung 133 oder Einschnürung oder Neigungsveränderung oder Verjüngung oder Asymmetrie der inneren und/oder äußeren Wandung des erfindungsgemäßen Luftabscheiders 100 auf. Die Einbuchtung 133 ist in Fig. 8 am rechten Rand und an der Vorderseite des erfindungsgemäßen Luftabscheiders 100 zu erkennen.

Die Einbuchtung 133 kann sich zumindest auf der der Zuströmleitung oder der dem Einströmkanal 9 gegenüber liegenden Seite befinden.

Die Einbuchtung 133 kann einen Abschnitt des Umfangs oder den gesamten Umfang des Hartteils der venösen Kammer betreffen.

Die Einbuchtung 133 kann im Wesentlichen horizontal liegen in einer Gebrauchsstellung der Blutkassette 1000.

Die Einbuchtung 133 kann in bestimmten Ausführungsformen einer Veränderung des Umfangs und/oder des Durchmessers eines Abschnitts des erfindungsgemäßen Luftabscheiders 100 oder der Wandung hiervon entsprechen oder diese umfassen.

Die Einbuchtung 133 kann in manchen Ausführungsformen auf die erste Kammer 3 beschränkt sein.

Die Einbuchtung 133 kann in manchen Ausführungsformen einen nicht-kreisrunden Durchmesser des erfindungsgemäßen Luftabscheiders 100 (in Höhe der Einbuchtung 133) in einem horizontalen Schnitt (bezogen auf die Darstellung der Blutbehandlungskassette 1000 in Fig. 8 oder auf eine Anordnung der Blutbehandlungskassette 1000 bei ihrem Gebrauch) bedeuten oder diesen umfassen.

Die Einbuchtung 133 kann in bestimmten Ausführungsformen zu einer asymmetrischen Form des erfindungsgemäßen Luftabscheiders 100 gemessen an der Darstellung der Fig. 8 in oben-unten-Richtung führen.

Die Einbuchtung 133, welche teilweise oder vollständig über den gesamten Querschnitt des erfindungsgemäßen Luftabscheiders 100 verlaufen kann, hat überraschender Weise zu einer verringerten Schaumbildung innerhalb des erfindungsgemäßen Luftabscheiders 100 geführt.

Bei der mittels der Einbuchtung 133 erzielten Optimierung der Geometrie des erfindungsgemäßen Luftabscheiders 100, welcher auch als eine venöse Blutkammer bezeichnet werden kann, mit welcher optional auch die Geometrie eines Single-Needle-Ventils optimiert worden sein kann, kann in bestimmten erfindungsgemäßen Ausführungsformen eine verbesserte Entlüftung vor Behandlungsbeginn erzielt werden. Ferner kann in manchen erfindungsgemäßen Ausführungsformen eine verbesserte Dampfströmung beim Sterilisieren erzielt werden. Zudem kann in bestimmten erfindungsgemäßen Ausführungsformen eine Verringerung von Totwasser-Gebieten mit den hiermit verbundenen, bekannten Vorteilen erzielt werden.

## Patentansprüche

1. Luftabscheider (100) für einen extrakorporalen Blutkreislauf zum Abscheiden von Luft aus einem den Luftabscheider (100) durchströmenden Fluid, aufweisend wenigstens eine Luftabscheidekammer (1) mit
- wenigstens einem Einströmkanal (9), durch den das Fluid in die Luftabscheidekammer (1) einströmen kann, und mit
- wenigstens einem Abströmkanal (25), durch welchen das Fluid aus der Luftabscheidekammer (1) ausströmen kann,
wobei die Luftabscheidekammer (1) aufweist:
- eine erste Kammer (3) mit wenigstens einem ersten Kammerquerschnitt (QK1), in welche der Einströmkanal (9) mündet,
- wenigstens eine zweite Kammer (5) mit einem zweiten Kammerquerschnitt (QK2), aus welcher der Abströmkanal (25) abgeht,
wobei
zwischen der ersten Kammer (3) und der zweiten Kammer (5) eine Verbindungsöffnung (7) vorgesehen ist, durch welche das Fluid aus der ersten Kammer (3) in die zweite Kammer (5) strömen kann,
wobei der erfindungsgemäße Luftabscheider (100) eine Einbuchtung (133) oder Einschnürung seiner inneren und/oder äußeren Wandung aufweist,
**dadurch gekennzeichnet, dass** die Einströmung des Fluids tangential zu wenigstens einer Seitenwand (17, 27) der ersten Kammer (3) erfolgt, und
dass der Einströmkanal (9) in einem unteren Bereich des Luftabscheiders (100) oder in einem unteren Bereich (11) der ersten Kammer (3) angeordnet ist..

2. Luftabscheider (100) nach Anspruch 1, wobei die erste Kammer (3) im Gebrauch des Luftabscheiders (100) oberhalb der zweiten Kammer (5) angeordnet ist.

3. Luftabscheider (100) nach Anspruch 2, wobei sich die Einbuchtung (133) zumindest auf der der Zuströmleitung oder der dem Einströmkanal (9) gegenüber liegenden Seite befindet.

4. Luftabscheider (100) nach einem der Ansprüche 2 oder 3, wobei sich die Einbuchtung (133) über einen Abschnitt des Umfangs oder den gesamten Umfang eines Hartteils des Luftabscheiders (100) erstreckt.

5. Luftabscheider (100) nach einem der Ansprüche 2 bis 4, wobei die Einbuchtung (133) in einer Gebrauchsstellung der Blutkassette (1000) im Wesentlichen horizontal liegt.

6. Luftabscheider (100) nach einem der Ansprüche 2 bis 5, wobei die Einbuchtung (133) auf die erste Kammer (3) beschränkt ist.

7. Luftabscheider (100) nach einem der vorangegangenen Ansprüche, wobei die Einströmung des Fluids unter einem Winkel von 0° bis 15°, bezogen auf eine Horizontale erfolgt.

8. Luftabscheider (100) nach einem der vorangegangenen Ansprüche, wobei sich ein Längsschnitt des Einströmkanals (9) zur ersten Kammer (3) hin aufweitet.

9. Luftabscheider (100) nach Anspruch 8, wobei der Querschnitt des Einströmkanals (9) kontinuierlich zunimmt.

10. Luftabscheider (100) nach Anspruch 8 oder 9, wobei ein Winkel der Aufweitung des Längsschnitts des Einströmkanals (9) zwischen 0° und 15° beträgt.

11. Externe Funktionseinrichtung, welche wenigstens einen Luftabscheider (100) gemäß einem der Ansprüche 1 bis 10 aufweist, wobei die externe Funktionseinrichtung eine Blutkassette ist.

12. Externe Funktionseinrichtung nach Anspruch 11, die als Spritzguss-Hartteil mit wenigstens einer aufgeschweißten Folie ausgestaltet ist.

13. Schlauchsystem zum extrakorporalen Führen von Blut, welches wenigstens einen Luftabscheider (100) gemäß einem der Ansprüche 1 bis 10 oder eine externe Funktionseinrichtung gemäß einem der Ansprüche 11 bis 12 aufweist.

14. Behandlungsvorrichtung, welche wenigstens einen Luftabscheider (100) gemäß einem der Ansprüche 1 bis 10 und/oder wenigstens eine externe Funktionseinrichtung gemäß einem der Ansprüche 11 bis 12 und/oder wenigstens ein Schlauchsystem gemäß Anspruch 13 aufweist, wobei die Behandlungsvorrichtung als Blutbehandlungsvorrichtung ausgestaltet ist.

## Claims

1. An air separator (100) for an extracorporeal blood circuit for separating air from a fluid flowing through the air separator (100), comprising at least one air separation chamber (1) having:
- at least one inlet channel (9), through which the fluid can flow into the air separation chamber (1), and having
- at least one outlet channel (25), through which the fluid can flow out of the air separation chamber (1),
wherein the air separation chamber (1) comprises:
- one first chamber (3) having at least one first chamber cross-section (QK1), into which the inlet channel (9) opens,
- at least one second chamber (5) having one second chamber cross-section (QK2), from which the outlet channel (25) leaves,
wherein
a connection opening (7) is provided between the first chamber (3) and the second chamber (5), through which the fluid can flow from the first chamber (3) into the second chamber (5),
wherein the air separator (100) according to the invention comprises an indentation (133) or a constriction of its inner and/or outer wall,
**characterized in that** the inlet of the fluid occurs tangentially to at least one side wall (17, 27) of the first chamber (3), and
**in that** the inlet channel (9) is arranged in a lower region of the air separator (100) or in a lower region (11) of the first chamber (3).

2. The air separator (100) according claim 1, wherein the first chamber (3) is arranged above the second chamber (5) during use the air separator (100).

3. The air separator (100) according claim 2, wherein the indentation (133) is located at least on the side opposite to the inflow line or the inlet channel (9).

4. The air separator (100) according to anyone of claims 2 or 3, wherein the indentation (133) extends over a portion of the periphery or the entire periphery of a hard part of the air separator (100).

5. The air separator (100) according to anyone of claims 2 to 4, wherein the indentation (133) lies substantially horizontally in a state of use of the blood cassette (1000) .

6. The air separator (100) according to anyone of claims 2 to 5, wherein the indentation (133) is limited to the first chamber (3).

7. The air separator (100) according to anyone of the preceding claims, wherein the inlet of the fluid occurs at an angle of 0° to 15° relative to a horizontal.

8. The air separator (100) according to anyone of the preceding claims, wherein a longitudinal section of the inlet channel (9) widens towards the first chamber (3).

9. The air separator (100) according to claim 8, wherein the cross-section of the inlet channel (9) increases continuously.

10. The air separator (100) according to claim 8 or 9, wherein an angle of widening of the longitudinal section of the inlet channel (9) ranges between 0° and 15°.

11. An external functional device comprising at least one air separator (100) according to anyone of claims 1 to 10, wherein the external functional device is a blood cassette.

12. The external functional device according to claim 11, which is designed as an injection-moulded hart part having at least one welded-on film.

13. A tube system for extracorporeal blood guidance, comprising at least one air separator (100) according to anyone of claims 1 to 10 or an external functional device according to anyone of claims 11 to 12.

14. A treatment apparatus, comprising at least one air separator (100) according to anyone of claims 1 to 10 and/or at least one external functional device according to anyone of claims 11 to 12 and/or at least one tube system according to claim 13, wherein the treatment apparatus is designed as a blood treatment apparatus.

## Revendications

1. Un séparateur d'air (100) pour un circuit sanguin extracorporel destiné à séparer l'air d'un fluide circulant à travers le séparateur d'air (100), comprenant au moins une chambre de séparation d'air (1) ayant:
- au moins un canal d'entrée (9), par lequel le fluide peut s'écouler dans la chambre de séparation d'air (1), et
- au moins un canal de sortie (25), par lequel le fluide peut s'écouler hors de la chambre de séparation d'air (1),
où la chambre de séparation d'air (1) comprend:
- une première chambre (3) ayant au moins une première section transversale de chambre (QK1), dans laquelle débouche le canal d'entrée (9),
- au moins une seconde chambre (5) ayant une seconde section transversale de chambre (QK2), de laquelle le canal de sortie (25) sort,
où
un orifice de liaison (7) est prévu entre la première chambre (3) et la seconde chambre (5), au travers duquel le fluide peut s'écouler de la première chambre (3) vers la seconde chambre (5),
où le séparateur d'air (100) selon l'invention comprend un évidement (133) ou un rétrécissement de sa paroi intérieure et/ou extérieure,
**caractérisé en ce que** l'entrée du fluide s'effectue tangentiellement à au moins une paroi latérale (17, 27) de la première chambre (3), et
**en ce que** le canal d'entrée (9) est agencé dans une région inférieure du séparateur d'air (100) ou dans une région inférieure (11) de la première chambre (3).

2. Le séparateur d'air (100) selon la première revendication, où la première chambre (3) est agencée au-dessus de la seconde chambre (5) lors de l'utilisation du séparateur d'air (100) .

3. Le séparateur d'air (100) selon la revendication 2, où l'évidement (133) est situé au moins du côté opposé au conduit d'arrivée ou au canal d'entrée (9).

4. Le séparateur d'air (100) selon l'une quelconque des revendications 2 ou 3, où l'évidement (133) s'étend sur une section de la périphérie, ou sur la totalité de la périphérie, d'une partie rigide du séparateur d'air (100).

5. Le séparateur d'air (100) selon l'une quelconque des revendications 2 à 4, où l'évidement (133) est essentiellement à l'horizontal lors de l'utilisation de la cassette à sang (1000).

6. Le séparateur d'air (100) selon l'une quelconque des revendications 2 à 5, où l'évidement (133) est limité à la première chambre (3).

7. Le séparateur d'air (100) selon l'une quelconque des revendications précédentes, où l'entrée du fluide s'effectue selon un angle de 0° à 15° par rapport à l'horizontal.

8. Le séparateur d'air (100) selon l'une quelconque des revendications précédentes, où une section longitudinale du canal d'entrée (9) s'élargit vers la première chambre (3).

9. Le séparateur d'air (100) selon la revendication 8, où la section transversale du canal d'entrée (9) augmente de façon continuelle.

10. Le séparateur d'air (100) selon la revendication 8 ou 9, où un angle d'élargissement du canal d'entrée (9) est compris entre 0° et 15°.

11. Un dispositif fonctionnel externe comprenant au moins un séparateur d'air (100) selon l'une quelconque des revendications 1 à 10, où le dispositif fonctionnel externe est une cassette à sang.

12. Le dispositif fonctionnel externe selon la revendication 11, qui est conçu comme une pièce rigide moulée par injection comprenant au moins un film soudé.

13. Un système de tuyaux pour le guidage extracorporel de sang, comprenant au moins un séparateur d'air (100) selon l'une quelconque des revendications 1 à 10 ou un dispositif fonctionnel externe selon l'une quelconque des revendications 11 à 12.

14. Un appareil de traitement, comprenant au moins un séparateur d'air (100) selon l'une quelconque des revendications 1 à 10 et/ou au moins un dispositif fonctionnel externe selon l'une quelconque des revendications 11 à 12 et/ou au moins un système de tuyaux selon la revendication 13, où l'appareil de traitement est conçu comme un appareil de traitement du sang.
